(19) Europäisches Patentamt
European Patent Office
Office européen des brevets

(11) **EP 2 850 485 B1**

(12) **FASCICULE DE BREVET EUROPEEN**

(45) Date de publication et mention de la délivrance du brevet:
**07.12.2022 Bulletin 2022/49**

(21) Numéro de dépôt: **13729973.1**

(22) Date de dépôt: **16.05.2013**

(51) Classification Internationale des Brevets (IPC):
*G02C 7/10* (2006.01)   *G02B 5/28* (2006.01)
*G02B 5/26* (2006.01)   *G02B 1/11* (2015.01)
*G02C 7/02* (2006.01)

(52) Classification Coopérative des Brevets (CPC):
**G02C 7/107; G02B 1/11; G02B 5/26; G02B 5/283; G02C 7/02; G02C 7/104**

(86) Numéro de dépôt international:
**PCT/FR2013/051075**

(87) Numéro de publication internationale:
**WO 2013/171435 (21.11.2013 Gazette 2013/47)**

(54) **LENTILLE OPHTALMIQUE**

BRILLENGLAS

OPHTHALMIC LENS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.05.2012 FR 1254529**
**11.10.2012 FR 1259713**

(43) Date de publication de la demande:
**25.03.2015 Bulletin 2015/13**

(73) Titulaire: **Essilor International**
**94220 Charenton-le-Pont (FR)**

(72) Inventeurs:
• **DE AYGUAVIVES, Francisco**
**F-94220 Charenton-le-Pont (FR)**
• **MAURY, Hélène**
**F-94220 Charenton-le-Pont (FR)**

(74) Mandataire: **Jacobacci Coralis Harle**
**32, rue de l'Arcade**
**75008 Paris (FR)**

(56) Documents cités:
**WO-A1-2012/076714      WO-A2-2008/024414**
**US-A1- 2004 233 524      US-A1- 2010 149 483**
**US-A1- 2012 075 577**

## Description

L'invention concerne le domaine de l'optique ophtalmique.

Elle concerne plus particulièrement une lentille ophtalmique comportant des moyens pour réduire à la fois la lumière ultraviolette et la lumière bleue, notamment la lumière bleue phototoxique arrivant sur la rétine d'un porteur de lunettes équipées de telles lentilles.

Dans l'ensemble de la présente demande de brevet, il sera fait référence à des gammes de valeurs, en particulier de longueurs d'onde et d'angles d'incidence. L'expression « compris entre les valeurs $x$ et $y$ » signifie « dans la gamme de $x$ à $y$ », les bornes $x$ et $y$ étant incluses dans cette gamme.

Comme cela est classique, on désignera par face avant, la face principale de la lentille la plus éloignée de l'œil d'un porteur et au contraire, par face arrière, la face principale de la lentille la plus proche de l'œil du porteur.

La lumière visible par l'œil humain s'étend sur un spectre lumineux allant d'une longueur d'onde de 380 nanomètres (nm) à 780 nm environ. La partie de ce spectre, située entre 380 nm et 500 nm environ, correspond à une lumière sensiblement bleue, de haute énergie.

La lumière ultraviolette (UV) constitue la partie du spectre lumineux inférieure à 380 nm et allant jusqu'à 100 nm. Les UVB correspondent à la gamme 280 nm à 320 nm et les UVA 320 à 380 nm.

De nombreuses études (voir par exemple Kitchel E., « The effects of blue light on ocular health », Journal of Visual Impairment and Blindness Vol. 94, No. 6, 2000 ou Glazer-Hockstein et al., Retina, Vol. 26, No. 1, pp. 1-4, 2006) suggèrent que la lumière bleue a des effets phototoxiques sur l'œil, et notamment sur la rétine.

En effet, des études de photobiologie oculaire (Algvere P.V. et al., « Age-Related Maculopathy and the Impact of the Blue Light Hazard », Acta Ophthalmo. Scand., Vol. 84, pp. 4-15, 2006) et des études cliniques (Tomany S.C. et al., « Sunlight and the 10-Year Incidence of Age-Related Maculopathy. The Beaver Dam Eye Study », Arch Ophthalmol. , Vol. 122, pp. 750-757, 2004) ont montré qu'une exposition à la lumière bleue trop longue ou trop intense peut induire des pathologies ophtalmiques sévères telles que la dégénérescence maculaire liée à l'âge (DMLA).

Néanmoins, une partie de cette lumière bleue, comprise entre 465 nm et 495 nm environ est bénéfique dans la mesure où elle intervient au niveau des mécanismes de régulation des rythmes biologiques, appelés « cycles circadiens ».

Ainsi, il est recommandé de limiter l'exposition à la lumière bleue potentiellement nocive, en particulier pour la bande de longueur d'onde qui présente une dangerosité accrue (voir notamment tableau B1 de la norme ISO 8980-3 :2003 (E) concernant la fonction de dangerosité de la lumière bleue $B(\lambda)$).

À cet effet, il peut être conseillé de porter devant chacun des yeux une lentille ophtalmique qui empêche ou limite la transmission de la lumière bleue phototoxique jusqu'à la rétine.

Il a déjà été proposé, par exemple dans les demandes de brevet US 2012/075577 ou WO2008024414, de couper au moins partiellement, la partie gênante du spectre de la lumière bleue de 400 nm à 460 nm au moyen de lentilles comprenant un film inhibant partiellement la lumière dans la gamme de longueurs d'onde souhaitée, par absorption ou par réflexion.

Des filtres coupant la lumière bleue sont également décrits dans le brevet US-8.360.574.

Il est en outre nécessaire d'éliminer le plus possible l'influence néfaste de la lumière ultraviolette (lumière UV) sur l'œil d'un porteur de la lentille.

Par ailleurs, l'homme du métier est à la recherche de filtres permettant de minimiser la quantité de lumière bleue nocive reçue par la rétine, tout en permettant de transmettre efficacement la lumière visible pour les longueurs d'onde supérieures à 465 nm afin, d'une part, de conserver une bonne vision pour le porteur et, d'autre part, de ne pas altérer les cycles circadiens.

La difficulté est que les gammes de longueurs d'onde de 420 nm à 450 nm qu'il convient de filtrer sont très proches des gammes de longueurs d'onde qui ne doivent pas, ou très peu, être filtrées.

La présente invention a donc pour objectif principal de fournir une lentille ophtalmique qui à la fois réduit efficacement la lumière UV et la lumière bleue reçues par un porteur de la lentille.

Dans le cas particulier où le moyen de blocage de la lumière est un filtre, notamment constitué par un revêtement antireflet de la lumière visible, une optimisation de ce filtre est souhaitable.

Un objectif plus spécifique de l'invention est de fournir une lentille ophtalmique comprenant un filtre par réflexion tenant compte de l'ensemble de l'irradiation lumineuse provenant du milieu environnant et réduisant la lumière bleue reçue par l'œil dans la gamme de longueurs d'onde allant de 420 nm à 450 nm.

Un autre objectif de l'invention est de fournir une lentille ophtalmique comprenant un tel filtre par réflexion qui permet une excellente transmission dans la gamme de longueurs d'onde allant de 465 nm à 495 nm.

Un autre objectif de l'invention est de fournir une lentille ophtalmique comportant des moyens de réduction de la lumière UV et de la lumière bleue reçues par un porteur de la lentille et notamment une lentille comportant un filtre par réflexion présentant les propriétés ci-dessus, qui soit facile et peu coûteux à mettre en œuvre industriellement.

Les buts ci-dessus sont atteints selon l'invention par une lentille ophtalmique présentant une face principale avant et une face principale arrière comprenant les caractéristiques définies dans la revendication 1.

De préférence, la lentille selon l'invention a un facteur de transmission pondéré dans le visible, $\tau_v$, d'au moins 90% (norme ISO 13266-1998).

Le moyen de coupure de la lumière UV arrivant sur la face principale avant de la lentille peut être constitué par le substrat de la lentille lui-même de par sa propre nature ou par incorporation et dispersion dans le matériau du substrat d'un ou plusieurs absorbeurs UV. Parmi les absorbeurs UV utilisables, on peut citer les oxanilides, les benzophénones, les dihydroxybenzophénones, les benzotriazoles, les benzoates, les phénylbenzoates, les benzimidazoles, les hydroxyphényltriazines et les amines à empêchement stérique (AES ou HALS). Ces absorbeurs UV sont disponibles commercialement notamment sous les dénominations UVINUL® (BASF), PARSOL® (GIVAUDAN).

Le moyen de coupure de la lumière UV peut aussi être un revêtement absorbant les UV ou un revêtement réfléchissant les UV disposé sur la face principale avant de la lentille ophtalmique.

Les revêtements absorbants les UV sont connus dans la technique. A titre d'exemple, on peut citer les films polymériques transparents contenant un ou plusieurs absorbeurs UV tels que ceux cités précédemment.

Les revêtements réfléchissant la lumière UV sont également connus. On peut utiliser à cette fin des revêtements antireflets classiques qui comme cela est connu, présentent une réflexion relativement importante ou importante dans le domaine des UV (280-380 nm) (voir l'article « Anti-reflecting coatings reflect ultraviolet radiation, Citek, K Optometry, 2008, 79, 143-148).

La réflexion moyenne dans les régions UVA et UVB peut atteindre des niveaux élevés (jusqu'à 60%) pour certains revêtements antireflets traditionnels. Typiquement, la réflexion moyenne dans l'UV des revêtements antireflets commercialement utilisés est de 10 à 25% pour un angle d'incidence de 30° et un angle d'incidence de 45°.

Bien évidemment, le moyen de coupure des UV peut être une combinaison quelconque des moyens décrits ci-dessus.

Comme indiqué ci-dessus, la lentille ophtalmique selon l'invention comporte, en face principale arrière, un revêtement antireflet ayant un facteur moyen pondéré de réflexion dans l'UV ($R_{UV}$) inférieur ou égal à 7%, de préférence inférieur ou égal à 6%, mieux inférieur ou égal à 5%, mieux encore inférieur ou égal à 4,5% et idéalement inférieur ou égal à 4%. D'autres valeurs optimales de facteur moyen pondéré de réflexion dans l'UV sont inférieures ou égales à 3,5% et mieux encore inférieures ou égales à 3%. Ce facteur moyen pondéré de réflexion dans la région ultraviolette, entre 280 et 380 nm, pour une incidence de 30° et une incidence de 45°, est donné par la relation :

$$R_{UV} = \frac{\int_{280}^{380} W(\lambda)R(\lambda).d\lambda}{\int_{280}^{380} W(\lambda).d\lambda}$$

où R($\lambda$) représente le facteur de réflexion spectral à une longueur d'onde donnée, et W($\lambda$) représente une fonction de pondération égale au produit de l'irradiance du spectre solaire Es($\lambda$) et la fonction d'efficacité relative spectrale S($\lambda$).

La fonction spectrale W($\lambda$) permet de calculer les facteurs de transmission du rayonnement ultraviolet et est définie selon la norme ISO 13666 :1998.

De tels revêtements antireflets sont décrits, entre autres, dans la demande de brevet internationale WO-2012/076714A1.

De préférence, le moyen de coupure de la lumière UV arrivant sur la face avant de la lentille coupe 90%, de préférence 95% ou plus, mieux encore 100% de la lumière UV incidente.

Selon l'invention, la lentille ophtalmique comporte encore, au moins un moyen bloquant, au moins en partie, la lumière bleue dans le domaine de longueur d'onde 420 à 450 nm.

De préférence, le moyen bloquant la lumière bleue bloque de 5 à 50%, de préférence 10 à 50%, mieux de 15 à 50% et de façon optimale de 20 à 50% de la lumière bleue dans le domaine de longueurs d'onde de 420 à 450 nanomètres.

Les moyens de réaliser un tel filtre bloquant la lumière bleue sont nombreux et connus dans la technique et comprennent des techniques d'absorption, réflexion, interférence, ou des combinaisons de ces techniques.

Selon une technique, la lentille peut être teintée/colorée avec un colorant de blocage de la lumière bleue tel que les produits BPI Filter Vision 450 ou BPI Diamond Dye 500, en proportion ou concentration appropriée. La coloration peut s'effectuer, par exemple, par immersion de la lentille dans un bain chaud contenant la solution de colorant de blocage pendant une durée prédéterminée.

Selon une autre technique, on utilise un filtre formé sur au moins une des faces principales de la lentille pour bloquer la lumière bleue.

Le filtre peut inclure, par exemple, des composés organiques ou inorganiques présentant une absorption et/ou une réflexion et/ou une interférence avec les longueurs d'onde de la lumière bleue. Le filtre peut être constitué de couches minces multiples de substances organiques et/ou inorganiques. Chaque couche peut avoir des propriétés qui, en combinaison avec les autres couches, absorbent, réfléchissent ou interfèrent avec les longueurs d'onde de la lumière bleue.

Parmi les substances bloquant la lumière bleue, on peut citer le pérylène, les molécules à base de porphyrine, coumarine et acrydine.

L'insertion de la substance bloquant la lumière bleue peut se faire par incorporation directe dans le substrat, addition à un revêtement polymérique, imbibation dans la lentille, incorporation dans une structure stratifiée comprenant une couche imprégnée de la substance, ou sous forme d'un matériau composite imprégné de microparticules de la substance.

Dans une réalisation recommandée de l'invention, le filtre bloquant la lumière bleue est constitué d'un empilement, éventuellement de type « rugate filter », de minces couches de matériau diélectrique, de préférence inorganique, ayant des indices de réfraction alternativement faibles et élevés, tels que $SiO_2$ et $TiO_2$. Les paramètres de conception tels que l'épaisseur de chaque couche mince, l'indice de réfraction de chaque couche et le nombre de couches déterminent les performances de l'empilement.

Des filtres de ce type sont décrits entre autres dans les brevets des Etats-Unis N° 6.984.038 et n° 7.066.596.

De manière générale, des filtres bloquant la lumière bleue sont décrits dans le brevet des Etats-Unis n° 8.360.574.

Le filtre bloquant la lumière bleue est un filtre, notamment un revêtement antireflet de la lumière visible, formé sur au moins une face principale de la lentille qui confère à cette face principale de la lentille la propriété suivante :

- pour un angle d'incidence $\theta$ compris entre 0° et 15° et pour un angle d'incidence $\theta'$ compris entre 30° et 45°, un paramètre $\Delta (\theta, \theta')$ défini par la relation $\Delta (\theta, \theta') = 1 - [R_\theta' (435 \text{ nm}) / R_\theta (435 \text{ nm})]$ tel que ce paramètre $\Delta (\theta, \theta')$ soit supérieur ou égal à 0,5, de préférence supérieur ou égal à 0,6, où

  ○ $R_\theta (435 \text{ nm})$ représente la valeur de la réflectivité de la face principale comportant ledit filtre à la longueur d'onde de 435 nanomètres pour l'angle d'incidence $\theta$, et
  ○ $R_\theta' (435 \text{ nm})$ représente la valeur de la réflectivité de la face principale comportant ledit filtre à la longueur d'onde de 435 nanomètres pour l'angle d'incidence $\theta'$.

De préférence, le filtre ci-dessus confère également à la face principale le comportant la propriété suivante :

- une courbe de réflectivité spectrale pour un angle d'incidence compris entre 0° et 15° ayant :

  ○ un maximum de réflectivité à une longueur d'onde inférieure à 435 nanomètres, et
  ○ une largeur à mi-hauteur (FWHM) supérieure ou égale à 70 nm, de préférence supérieure ou égale à 75 nm et mieux supérieure ou égale à 80 nm et inférieure à 150 nm, de préférence inférieure ou égale à 120 nm et mieux inférieure ou égale à 110 nm.

De préférence, la lentille ophtalmique selon l'invention est conçue de telle sorte que le facteur moyen de réflexion lumineux dans le visible ($R_v$) sur chacune des faces principales de la lentille ophtalmique est ou égal à 3%, mieux inférieur ou égal à 2,5%, mieux encore inférieur ou égal à 2% et de préférence inférieur ou égal à 1,5%, et de façon optimale inférieur ou égal à 1%.

La lentille ophtalmique selon l'invention peut encore comporter un composant «d'équilibrage de la couleur » afin de réduire, déplacer, neutraliser ou compenser de toute manière le jaunissement ou la couleur ambrée, ou tout autre effet indésirable dû au blocage de la lumière bleue afin de produire une lentille cosmétiquement acceptable.

Ainsi, le composant peut être un revêtement formé sur une face principale de la lentille, généralement la face opposée à celle comportant le moyen de blocage de la lumière bleue, traité avec un ou plusieurs additifs d'équilibrage de la couleur, par exemple une combinaison appropriée de colorants rouges et verts. Le composant d'équilibrage de la couleur peut être un film mono ou multicouches tel qu'un revêtement antireflet ou un revêtement anti-abrasion.

L'équilibrage de la couleur dans la lentille peut se faire à la fois pour la lumière transmise et pour la lumière réfléchie.

Les composants d'équilibrage de la lumière sont décrits en particulier dans le brevet des Etats-Unis n° 8.360.574.

La lentille peut avoir un indice de jaune élevé. Celui-ci sera d'autant plus élevé que le filtre bloquera une partie importante de la lumière bleue, en l'absence de composants d'équilibrage de la couleur.

Les valeurs de Yi mesurées conformément à la norme ASTM E 313 05 peuvent donc être élevées (supérieures à 50). De préférence, les valeurs de Yi sont inférieures ou égales à 50, 40, 35, 30, 25, 23, 20, 15, 10, 9, 7 ou 5.

Dans une réalisation recommandée, la lentille ophtalmique selon l'invention constitue un verre de lunette, notamment un verre correcteur, ce verre de lunette pouvant être un verre blanc, un verre teinté, un verre solaire ou un verre photochromique.

D'une manière générale, il est possible de concevoir des filtres, dits « étroits », très sélectifs, présentant une bande passante limitée et un pic de réflectivité centré sur cette bande passante. Pour limiter la transmission de la lumière bleue phototoxique à la rétine, un filtre étroit adéquat devrait ainsi avoir par exemple une largeur à mi-hauteur de 30 nm entre 420 nm et 450 nm, et un maximum de réflectivité pour la longueur d'onde centrale de 435 nm.

En pratique, des filtres étroits très sélectifs sont généralement constitués d'un empilement comportant un nombre important de couches diélectriques et dont l'épaisseur totale est élevée.

De tels filtres sont longs et coûteux à réaliser industriellement, en particulier lorsque ceux-ci sont déposés sous vide. La multiplication du nombre de couches et la multiplication des interfaces rend également difficile d'atteindre de bonnes propriétés mécaniques.

La prise en compte des contraintes ci-dessus amène à limiter le nombre de couches, ce qui a pour conséquence de limiter les performances en termes de sélectivité spectrale (la largeur à mi-hauteur d'un tel filtre étroit pouvant alors atteindre jusqu'à 70 nm) et angulaire, le filtre devenant faiblement sélectif angulairement. Ceci signifie que si, pour des longueurs d'onde comprises entre 420 nm et 450 nm, la réflectivité de la face principale d'une lentille ophtalmique munie d'un tel filtre étroit est élevée pour des angles d'incidence sur cette face principale compris entre 0° et 15°, alors la réflectivité pour des angles d'incidence compris entre 30° et 45° sur cette même face principale sera également relativement élevée.

L'angle d'incidence est classiquement défini comme l'angle entre la normale à la surface au point d'incidence et la direction du faisceau lumineux contactant cette surface.

Ceci entraîne un certain nombre de conséquences pour un porteur de lunettes équipé de lentilles ophtalmiques sur la face principale avant desquelles a été déposé un filtre optique étroit tel que décrit précédemment. On entendra ici que la face principale avant de la lentille ophtalmique est la face principale de la lentille ophtalmique qui est située le plus loin des yeux du porteur de lunettes. Par opposition, on désignera la face principale de la lentille ophtalmique qui est située le plus près des yeux du porteur la face principale arrière.

Ainsi positionnées par rapport aux yeux du porteur, les lentilles ophtalmiques reçoivent, d'une part, de la lumière « directe » incidente sur les faces principales avant des lentilles ophtalmiques, et, d'autre part, de la lumière « indirecte » provenant de l'arrière du porteur et réfléchie par les lentilles ophtalmiques.

La lumière venant de l'arrière du porteur et réfléchie par la lentille ophtalmique en direction de l'œil du porteur est principalement la lumière incidente sur la face principale arrière de la lentille ophtalmique avec des angles d'incidence compris entre 30° et 45°.

Cette lumière visible provenant de l'arrière du porteur à un angle d'incidence compris entre 30° et 45° traverse la face principale arrière, sur laquelle se produit une première réflexion, puis le substrat pour atteindre la face principale avant comportant ledit filtre.

Par ailleurs, il est connu que les propriétés optiques d'un filtre déposé sur la face principale avant d'une lentille ophtalmique, par exemple la réflectivité, sont équivalentes, que la lumière soit incidente du côté de la face principale avant ou provenant du côté de la face principale arrière.

Si le filtre étroit réfléchit efficacement la lumière bleue dont la longueur d'onde est comprise entre 420 nm et 450 nm pour un angle d'incidence sur la face principale avant compris entre 30° et 45°, alors il réfléchit également efficacement cette lumière bleue arrivant par l'arrière, pour un angle d'incidence compris entre 30° et 45° sur la face principale arrière.

Ainsi, même si la lumière directe incidente sur les faces principales avant des lentilles ophtalmiques est rejetée efficacement grâce à la réflexion sur les filtres étroits déposés sur les faces principales avant, la lumière indirecte provenant de l'arrière du porteur est réfléchie tout aussi efficacement vers les yeux du porteur.

Au final, malgré l'utilisation d'un filtre étroit, la quantité de lumière bleue phototoxique parvenant jusqu'à la rétine du porteur pourra être relativement importante et présenter un danger pour le porteur.

Par ailleurs, le filtre, qu'il soit appliqué en face avant ou arrière se comporte de la même façon vis-à-vis de la lumière dans la gamme de longueur d'ondes de 420 nm à 450 nm, puisque la lentille ophtalmique, dans l'un comme dans l'autre des cas, transmet la lumière dans la gamme de longueurs d'onde de 420 nm à 450 nm. La même conséquence néfaste vis-à-vis de la lumière bleue phototoxique se manifeste donc pour le porteur si le filtre, au lieu d'être appliqué sur la face principale avant de la lentille ophtalmique, est appliqué sur la face principale arrière.

Par ailleurs, comme déjà mentionné précédemment, des filtres par réflexion étroits comportant un nombre restreint de couches et une épaisseur compatible avec une fabrication industrielle à grande échelle ne présentent qu'une sélectivité spectrale réduite, et sont susceptibles de réfléchir une partie significative de la lumière dans la gamme régissant les cycles circadiens.

Afin de répondre aux objectifs de l'invention et de remédier aux inconvénients précités de l'état de la technique, la présente invention propose une lentille ophtalmique munie d'un filtre par réflexion permettant de réduire la quantité de la lumière bleue phototoxique arrivant sur la rétine d'un porteur de cette lentille ophtalmique, tout en préservant au mieux les cycles circadiens.

Ainsi, la lentille ophtalmique selon l'invention permet de minimiser la transmission de la lumière bleue phototoxique à la rétine d'un porteur de cette lentille ophtalmique, grâce, d'une part, à sa réflectivité moyenne sur une gamme de longueurs d'onde allant de 420 nm à 450 nanomètres et grâce d'autre part, à sa sélectivité angulaire.

En effet, la lentille ophtalmique munie dudit filtre présente, à une longueur d'onde donnée, une réflectivité sensiblement différente pour deux angles d'incidence sur la face principale comportant ledit filtre sensiblement différents.

Par ailleurs, ce filtre est décentré par rapport à la bande de longueurs d'onde de la lumière bleue phototoxique comprise entre 420 nanomètres et 450 nanomètres. En effet, la lentille ophtalmique présente un maximum de réflectivité à une longueur d'onde inférieure à 435 nanomètres. Ceci permet alors d'ajuster la sélectivité angulaire de la lentille.

Les caractéristiques spectrales (réflectivité, $R_m$, $R_v$,..) de chaque face principale d'une lentille ophtalmique selon l'invention sont déterminées de façon classique pour un faisceau lumineux incident arrivant sur la face principale depuis l'air, sans avoir traversé le substrat.

Enfin, une lentille ophtalmique selon l'invention présentant un paramètre $\Delta(\theta,\theta')$ tel que défini ci-dessus permet :

- de maximiser la réflexion de la lumière bleue phototoxique provenant du côté de la face principale avant, l'intensité de cette réflexion étant liée à la grandeur $R_\theta(435\ nm)$, et
- de minimiser la réflexion de la lumière bleue phototoxique provenant du côté de la face principale arrière, l'intensité de cette réflexion étant liée à la grandeur $R_{\theta'}(435\ nm)$.

Ainsi, la lentille ophtalmique selon l'invention, munie de son filtre, réduit la transmission globale de la lumière bleue phototoxique vers la rétine d'un porteur d'une telle lentille ophtalmique.

Le filtre proposé présentant une largeur à mi-hauteur plus importante qu'un filtre étroit, il s'avère moins épais qu'un tel filtre étroit et comprend moins de couches, et par conséquent est de fabrication plus aisée et moins coûteuse qu'un filtre étroit.

Par ailleurs, d'autres caractéristiques avantageuses et non limitatives de la lentille ophtalmique selon l'invention sont les suivantes :

- le filtre est formé sur la face principale avant de la lentille ophtalmique ;
- le paramètre $\Delta(\theta,\theta')$ est défini pour un angle d'incidence $\theta$ sur la face principale comportant ledit filtre tel que $\theta = 15°$ et pour un angle d'incidence $\theta'$ sur la face principale comportant ledit filtre tel que $\theta' = 45°$ ;
- le paramètre $\Delta_{spectral}$ est défini pour un angle d'incidence de 15° ;
- le paramètre $\Delta(\theta,\theta')$ est supérieur ou égal à 0,65, mieux supérieur ou égal à 0,7, mieux encore supérieur ou égal à 0,75, et de façon optimale supérieur ou égal à 0,8 ;
- le maximum de réflectivité est à une longueur d'onde inférieure ou égale à 410 nm, mieux inférieure ou égale à 400 nm, et encore mieux, inférieure ou égale à 390 nm ;
- le maximum de réflectivité est à une longueur d'onde supérieure ou égale à 350 nanomètres, de préférence dans la gamme de longueurs d'onde allant de 360 nm à 400 nm, mieux dans la gamme de longueurs d'onde allant de 370 nm à 390 nm ;
- la largeur à mi-hauteur est supérieure ou égale à 90 nanomètres, de préférence supérieure ou égale à 100 nanomètres ;
- la largeur à mi-hauteur est inférieure ou égale à 150 nanomètres, de préférence inférieure ou égale à 120 nanomètres, mieux inférieure ou égale à 110 nm.

Ainsi, la largeur à mi-hauteur est généralement comprise entre 80 nm et 150 nm, de préférence entre 90 nm et 120 nm, mieux entre 90 nm et 110 nm et mieux encore entre 100 nm et 110 nm.

Enfin, d'autres caractéristiques avantageuses et non limitatives de la lentille ophtalmique selon l'invention sont les suivantes :

- la valeur de la réflectivité au maximum de réflectivité de la face principale comportant le filtre, pour un angle d'incidence de 15°, est de préférence au moins 1,5 fois supérieure, mieux au moins 2 fois supérieure et de façon optimale au moins 2,5 fois supérieure à la valeur de la réflectivité de cette même face principale, pour le même angle d'incidence, et à la longueur d'onde de 435 nm ;
- le ratio $[R_{15°}(435\ nm)-R_{15°}(480nm)]/R_{15°}(435nm)$ où $R_{15°}(435nm)$ et $R_{15°}(480nm)$ représentent respectivement la réflectivité de la face principale de la lentille ophtalmique comportant ledit filtre à une longueur d'onde de 435 nm et à une longueur d'onde de 480 nm, pour un angle d'incidence sur cette face principale de 15°, est supérieur ou égal à 0,8, mieux supérieur ou égal à 0,85 et mieux encore supérieur ou égal à 0,9. Ce ratio caractérise l'excellente sélectivité du filtre équipant la lentille ophtalmique selon l'invention qui permet de protéger de la bande phototoxique sans perturbation de la bande chronobiologique ;
- le facteur moyen de réflexion lumineux ($R_v$) sur la face principale de la lentille ophtalmique comportant le filtre est inférieur ou égal à 2,5%, de préférence inférieur ou égal à 1,5% ;

- le facteur moyen de réflexion lumineux ($R_v$) sur chacune des faces principales de la lentille ophtalmique est inférieur ou égal à 2,5%, de préférence inférieur ou égal à 1,5% ;
- le facteur moyen de réflexion lumineux ($R_v$) sur la face principale de la lentille ophtalmique comportant le filtre est inférieur ou égal à 0,7% ;
- le filtre est formé sur la face principale avant de la lentille ophtalmique et le facteur moyen de réflexion (non pondéré) dans la gamme ultraviolet (UV) allant de 300 nm à 380 nm, pour un angle d'incidence sur cette face principale avant de 15°, est supérieur ou égal à 15%, mieux supérieur ou égal à 20% et mieux encore supérieur ou égal à 25% ;
- le filtre est un filtre interférentiel ;
- le filtre comporte un nombre de couches inférieur ou égal 11, préférentiellement de 2 à 10 couches, et encore plus préférentiellement de 4 à 9 couches et mieux encore de 4 à 7 couches ;
- le filtre présente une épaisseur totale inférieure ou égale à 700 nanomètres, de préférence inférieure ou égale à 600 nanomètres, et mieux encore inférieure ou égale à 550 nm, et de façon optimale de 200 nm à 400 nm ;
- la face principale arrière de la lentille ophtalmique comporte un revêtement anti-UV, c'est-à-dire réfléchissant peu les UV, préférentiellement un revêtement antireflet efficace dans les UV et dans le visible.

En outre, la lentille ophtalmique selon l'invention entre avantageusement dans la réalisation de paire de lunettes.

Ainsi, l'invention propose également une paire de lunettes comportant au moins une lentille ophtalmique selon l'invention.

L'utilisation d'une lentille ophtalmique selon l'invention permet d'accroître le contraste de vision d'un porteur. L'utilisation de lentilles selon l'invention permet ainsi d'améliorer le confort de vision du porteur, et notamment de reconnaître plus facilement les objets ou personnes observés au travers de ladite lentille ophtalmique. Cette utilisation trouvera son intérêt pour tout sujet, en particulier pour un sujet sain ne présentant pas de pathologies oculaires ou de prédisposition à une telle pathologie.

Par ailleurs, l'utilisation d'une lentille ophtalmique selon l'invention s'avère particulièrement intéressante pour un usage thérapeutique ou pour prévenir les maladies liées à la phototoxicité de la lumière bleue.

L'utilisation d'une lentille ophtalmique selon l'invention permet de réduire les risques d'apparition d'une pathologie ophtalmique due à un processus dégénératif due à la photo-toxicité de la lumière bleue.

L'utilisation d'une lentille ophtalmique selon l'invention permet enfin de protéger de la phototoxicité de la lumière bleue au moins une partie de l'œil d'un porteur, en particulier pour le protéger d'un processus dégénératif tel que la dégénérescence maculaire liée à l'âge (DMLA).

Une réalisation préférentielle de l'invention sera décrite plus en détail en référence aux dessins annexés, dans lesquels des lentilles ophtalmiques présentent un filtre de la lumière bleue selon l'invention sur leur face principale avant :

- les figures 1 à 3 présentent les courbes de réflectivité spectrale pour un angle d'incidence sur la face principale avant de 15°, des lentilles ophtalmiques préparées dans les exemples 1 à 3 de la présente demande.
- la figure 4 présente pour chacun des exemples précédents le facteur de transmission pondéré (en %) et la réflectivité arrière pondérée (en %), la pondération étant faite au moyen de la fonction de risque de la lumière bleue.
- la figure 5 représente les courbes de réflectivité spectrale entre 380 nm et 500 nm de la lentille ophtalmique de l'exemple 3 de la présente demande pour des angles d'incidence sur la face principale avant de 0° et 45°.

Comme cela est bien connu, la lentille ophtalmique selon l'invention comprend un substrat transparent en verre minéral ou organique. Ce substrat peut comprendre un ou plusieurs revêtements fonctionnels pour conférer à la lentille ophtalmique des propriétés optiques et/ou mécaniques particulières, tels que, par exemple, un revêtement anti-choc, un revêtement anti-abrasion, un revêtement anti-reflet, un revêtement anti-UV, un revêtement anti-statique, un revêtement polarisant, et un revêtement anti-salissure et/ou anti-buée. Tous ces revêtements sont bien connus dans la technique des lentilles ophtalmiques.

Le substrat de la lentille ophtalmique selon l'invention est de préférence en verre organique, par exemple une matière plastique thermoplastique ou thermodurcissable.

Parmi les matériaux thermoplastiques convenant pour les substrats, on peut citer les (co)polymères (méth)acryliques, en particulier le poly(méthacrylate de méthyle) (PMMA), les (co)polymères thio(méth)acryliques, le polyvinylbutyral (PVB), les polycarbonates (PC), les polyuréthanes (PU), les poly(thiouréthanes), les (co)polymères d'allylcarbonates de polyols, les copolymères thermoplastiques éthylène/acétate de vinyle, les polyesters tels que le poly(téréphtalate d'éthylène) (PET) ou le poly(téréphtalate de butylène) (PBT), les polyépisulfures, les polyépoxydes, les copolymères polycarbonates/polyesters, les copolymères de cyclo-oléfines tels que les copolymères éthylène/norbornène ou éthylène/cyclopentadiène et leurs combinaisons.

Par (co)poiymère, on entend un copolymère ou un homopolymère. Par (méth)acrylate, on entend un acrylate ou un méthacrylate. Par polycarbonate (PC), on entend au sens de la présente invention aussi bien les homopolycarbonates que les copolycarbonates et les copolycarbonates séquencés.

Les substrats particulièrement recommandés sont les substrats obtenus par (co)polymérisation du bis allyl carbonate du diéthylèneglycol, vendu, par exemple, sous la dénomination commerciale CR-39® par la société PPG Industries (lentilles ORMA® ESSILOR), ou par polymérisation des monomères thio(méth)acryliques, tels que ceux décrits dans la demande de brevet français FR 2734827. Les substrats peuvent être obtenus par polymérisation de mélanges des monomères ci-dessus, ou peuvent encore comprendre des mélanges de ces polymères et (co)polymères.

D'autres substrats préférés sont les polycarbonates.

La lentille ophtalmique présente une face principale avant et une face principale arrière.

Par face principale arrière, on entend la face principale qui, lors de l'utilisation de la lentille ophtalmique, est la plus proche de l'œil de l'utilisateur. Il s'agit généralement d'une face concave. Inversement, par face principale avant, on entend la face principale qui, lors de l'utilisation de la lentille ophtalmique, est la plus éloignée de l'œil de l'utilisateur. Il s'agit généralement d'une face convexe.

Selon l'invention, l'une au moins des faces principales de la lentille ophtalmique comporte un filtre.

Comme indiqué précédemment, le substrat de la lentille ophtalmique peut comporter différents revêtements soit sur la face principale avant de la lentille ophtalmique, soit sur la face principale arrière de la lentille ophtalmique.

Un revêtement qui est "sur" le substrat ou qui a été déposé "sur" le substrat est défini comme un revêtement qui :

(i) est positionné au-dessus d'une face principale du substrat,

(ii) n'est pas nécessairement en contact avec le substrat, c'est-à-dire qu'un ou plusieurs revêtements intermédiaires peuvent être disposés entre le substrat et le revêtement en question, et

(iii) ne recouvre pas nécessairement complètement la face principale du substrat.

Lorsque "une couche A est localisée sous une couche B", on comprendra que la couche B est plus éloignée du substrat que la couche A.

Dans un mode de réalisation, le filtre est formé directement sur la face principale avant de la lentille ophtalmique.

Dans un autre mode préféré, il est déposé directement sur un revêtement anti-abrasion et/ou anti-rayures ayant lui-même été déposé sur la face principale avant de la lentille ophtalmique.

Avant le dépôt du filtre, il est courant de soumettre la surface dudit substrat, à un traitement d'activation physique ou chimique, destiné à augmenter l'adhésion du filtre sur la ou les faces principales.

Ce prétraitement est généralement conduit sous vide. Il peut s'agir d'un bombardement avec des espèces énergétiques, par exemple un faisceau d'ions (« Ion Pre-Cleaning » ou "IPC") ou un faisceau d'électrons, d'un traitement par décharge corona, par effluvage, d'un traitement UV, ou d'un traitement par plasma sous vide, généralement un plasma d'oxygène ou d'argon. Il peut également s'agir d'un traitement de surface acide ou basique et/ou par solvants (eau ou solvant organique).

Dans la présente demande, la réflectivité spectrale de la lentille ophtalmique, pour un angle d'incidence donné de la face comportant ledit filtre, représente la variation de la réflectivité (*i.e.* du facteur de réflexion) à cet angle d'incidence en fonction de la longueur d'onde. La courbe de réflectivité spectrale correspond à une représentation graphique de la réflectivité spectrale dans laquelle on trace la réflectivité spectrale (ordonnées) en fonction de la longueur d'onde (abscisses). Les courbes de réflectivité spectrales peuvent être mesurées au moyen d'un spectrophotomètre, par exemple un spectrophotomètre Perkin Elmer Lambda 850 équipé URA (Universal Reflectance Accessory),

Le facteur moyen de réflexion, noté $R_m$, est tel que défini dans la norme ISO 13666:1998, et mesuré conformément à la norme ISO 8980-4 (à un angle d'incidence inférieur à 17°, typiquement de 15°), c'est-à-dire qu'il s'agit de la moyenne (non pondérée) de la réflectivité spectrale sur l'ensemble du spectre lumineux allant de 400 nm à 700 nm.

De même, le facteur de réflexion lumineux, noté $R_v$, également appelé dans la présente demande facteur moyen de réflexion lumineux est tel que défini dans la norme ISO 13666:1998, et mesuré conformément à la norme ISO 8980-4 (à un angle d'incidence inférieur à 17°, typiquement de 15°), c'est-à-dire qu'il s'agit de la moyenne pondérée de la réflectivité spectrale sur l'ensemble du spectre lumineux visible compris entre 380 nm et 780 nm.

Par analogie, on définit un facteur moyen de réflexion dans le bleu entre 420 nm et 450 nm, noté $R_{m,B}$, qui correspond à la moyenne (non pondérée) de la réflectivité spectrale sur la gamme de longueurs d'onde allant de 420 nm à 450 nm.

Le facteur moyen de réflexion dans le bleu $R_{m,B}$ peut être mesuré pour un angle d'incidence sur la face principale comportant le filtre compris entre 0° (incidence normale) et 15°, préférentiellement à 15°.

Dans la présente demande, on notera également :

-   $R_\theta$(435 nm) la valeur de la réflectivité de la face principale de la lentille ophtalmique comportant le filtre selon l'invention, cette valeur étant déterminée (par mesure ou par calcul) à une longueur d'onde de 435 nanomètres et pour un angle d'incidence θ sur la face principale comportant le filtre compris entre 0° et 15°, et
-   $R_{\theta'}$(435 nm) la valeur de la réflectivité de la face principale de la lentille ophtalmique comportant le filtre selon l'invention, cette valeur étant déterminée (par mesure ou par calcul) à une longueur d'onde de 435 nanomètres et pour un angle d'incidence θ' sur la face principale comportant le filtre compris entre 30° et 45°.

On définira alors un paramètre Δ(θ,θ') par la relation suivante: Δ(θ,θ')=1-[R_{θ'}(435nm)/R_θ(435nm)]. On verra dans la suite de la description comment ce paramètre Δ(θ,θ') permet d'évaluer l'efficacité d'une lentille ophtalmique à limiter la quantité de lumière bleue phototoxique arrivant sur la rétine d'un porteur, en tenant compte des contributions respectives de la lumière bleue provenant du côté de la face avant ou du côté de la face arrière de la lentille.

Conformément à l'invention, le filtre confère à la face principale de la lentille ophtalmique comprenant le filtre la propriété de présenter, pour un angle d'incidence sur cette face principale compris entre 0° et 15°, un facteur moyen de réflexion dans le bleu $R_{m,B}$ qui est supérieur ou égal à 5%.

Le filtre est ainsi spécialement conçu pour maximiser le facteur moyen de réflexion dans le bleu $R_{m,B}$. Ceci permet de maximiser la réjection de la lumière bleue phototoxique, dans la gamme de longueurs d'onde allant de 420 nm à 450 nm, arrivant de manière directe sur la face principale avant de la lentille. On considère ici que la majeure partie de la lumière directe qui provient de l'avant du porteur de la lentille ophtalmique et qui atteint la rétine de celui-ci présente une incidence faible sur la face principale avant, généralement comprise entre 0° et 15°.

Selon un autre exemple, le facteur moyen de réflexion dans le bleu $R_{m,B}$, pour un angle d'incidence sur la face principale de la lentille ophtalmique comportant le filtre compris entre 0° et 15°, de préférence 15°, est supérieur ou égal à 10%, mieux supérieur ou égal à 20%, et encore mieux supérieur ou égal à 30%, et de façon optimale supérieur ou égal à 50%.

Conformément à l'invention, le filtre peut conférer également à la face principale comportant le filtre la propriété de présenter une courbe de réflectivité spectrale pour un angle d'incidence sur cette face principale compris entre 0° et 15°, de préférence 15°, qui a :

- un maximum de réflectivité à une longueur d'onde inférieure à 435 nanomètres, et
- une largeur à mi-hauteur (FWHM) supérieure ou égale à 80 nanomètres.

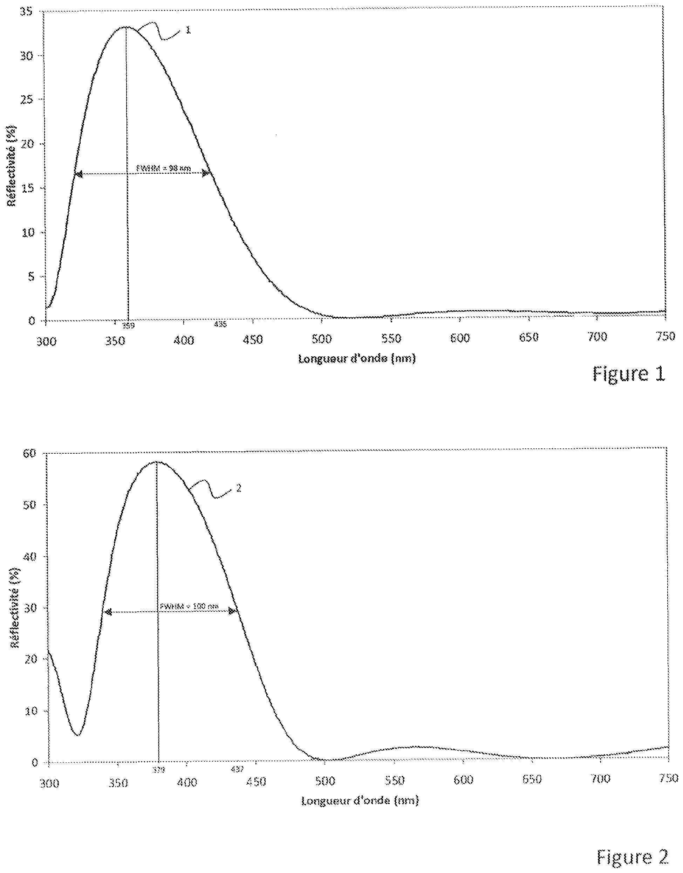

En effet, comme cela est visible sur les figures 1 à 3, les courbes de réflectivité spectrale de la face principale avant des lentilles ophtalmiques conformes à l'invention, présentent généralement, dans la gamme de longueurs d'onde allant de 380 nm à 500 nm, une forme de « cloche » que l'on peut caractériser par sa hauteur (maximum de réflectivité) et sa largeur à mi-hauteur (FWHM ou *« Full Width at Half Maximum »* en anglais).

Selon l'invention, le maximum de réflectivité est obtenu pour une longueur d'onde inférieure à 435 nm. Il est donc décalé par rapport à la longueur d'onde centrale (435 nm) de la bande de longueurs d'onde comprises entre 420 nm et 450 nm de la lumière bleue phototoxique.

De préférence, le maximum de réflectivité est à une longueur d'onde inférieure ou égale à 410 nm, mieux inférieure ou égale à 400 nm et encore mieux inférieure ou égale à 390 nm.

Dans un mode de réalisation préféré, ce décalage est limité de telle sorte que le maximum de réflectivité soit également à une longueur d'onde supérieure ou égale à 350 nm. De préférence, le maximum de réflectivité est à une longueur d'onde supérieure à 360 nm, mieux supérieure ou égale à 370 nm.

Selon l'invention, la largeur à mi-hauteur de la courbe de réflectivité spectrale considérée, pour un angle d'incidence sur la face principale comportant le filtre compris entre 0° et 15° est supérieure ou égale à 80 nm.

Un filtre dimensionné de telle sorte que la courbe de réflectivité spectrale pour un angle d'incidence sur la sur la face principale comportant le filtre compris entre 0° et 15° ait une largeur à mi-hauteur (FWHM) supérieure ou égale à 80 nanomètres, sera désigné par la suite filtre large.

Dans un mode de réalisation préférée, la largeur à mi-hauteur est supérieure ou égale à 90 nanomètres, de préférence supérieure ou égale à 100 nanomètres.

De préférence également, la largeur à mi-hauteur est inférieure à 150 nanomètres, mieux inférieure à 120 nanomètres, mieux encore inférieure à 110 nm.

Toujours selon l'invention, le filtre confère enfin à la face principale de la lentille ophtalmique comportant le filtre la propriété de présenter un paramètre Δ(θ,θ'), tel que défini précédemment, supérieur ou égal à 0,6.

Tel que défini précédemment, le paramètre Δ(θ,θ') dépend à la fois de la réflectivité à 435 nm pour un angle d'incidence θ sur la face principale compris entre 0° et 15°, notée $R_θ$(435 nm) et à la fois de la réflectivité à 435 nm pour un angle d'incidence θ' sur la face principale compris entre 30° et 45°, notée $R_{θ'}$(435 nm).

Dans le cas d'un lentille ophtalmique selon l'invention, placée devant l'œil d'un porteur, comme expliqué en introduction, on comprend que la quantité de lumière bleue phototoxique comprise dans la gamme de longueurs d'onde allant de 420 nm à 450 nm arrivant de manière directe sur la face principale avant de la lentille ophtalmique et parvenant à l'œil du porteur varie dans le sens inverse de la grandeur $R_θ$(435 nm).

De la même manière, la quantité de lumière bleu phototoxique comprise dans la gamme de longueurs d'onde allant de 420 nm à 450 nm arrivant indirectement de l'arrière du porteur et réfléchie par la lentille ophtalmique varie dans le même sens que la grandeur $R_{θ'}$(435 nm).

Ainsi, en choisissant un paramètre $\Delta(\theta,\theta')$ tel que $\Delta(\theta,\theta') \geq 0,6$, on obtient une lentille ophtalmique avec un filtre efficace et optimisé contre la lumière bleue phototoxique. En effet, le paramètre $\Delta(\theta,\theta')$ est d'autant plus élevé que :

(i) la valeur de la réflectivité $R_{\theta'}(435\ nm)$ est faible, c'est-à-dire que la quantité de lumière bleue phototoxique venant de l'arrière du porteur et réfléchie par la lentille ophtalmique en direction de la rétine du porteur est faible, et que
(ii) la valeur de la réflectivité $R_{\theta}(435\ nm)$ est élevée, c'est-à-dire que la quantité de lumière bleue phototoxique arrivant de manière directe sur la face principale avant de la lentille ophtalmique et réfléchie par celle-ci est élevée.

Dans un mode de réalisation préféré, le paramètre $\Delta(\theta,\theta')$ de la lentille ophtalmique munie d'un filtre large selon l'invention est supérieur ou égal à 0,7, mieux supérieur ou égal à 0,75, et mieux encore supérieur ou égal à 0,8.

De préférence, le paramètre $\Delta(\theta,\theta')$ est déterminé pour un angle d'incidence $\theta$ sensiblement égal à 15° et un angle d'incidence $\theta'$ sensiblement égal à 45°.

De préférence, le facteur moyen de transmission dans le bleu entre 465 nm et 495 nm de la lentille ophtalmique selon l'invention (pour un angle d'incidence sur la face principale avant compris entre 0° et 15°), qui correspond à la moyenne (non pondérée) de la transmitivité spectrale sur la gamme de longueurs d'onde allant de 465 nm à 495 nm, est supérieur ou égal à 80%, mieux supérieur ou égal à 85%, et encore mieux supérieur ou égal à 90%.

Ceci permet notamment de garantir que la majeure partie de la lumière bleue comprise entre 465 nm et 495 nm, qui serait responsable de la synchronisation de l'horloge biologique, soit transmise à l'œil d'un porteur équipé de cette lentille ophtalmique.

De manière préférée, le facteur de transmission de la lentille ophtalmique à 480 nm pour un angle d'incidence sur la face principale avant compris entre 0° et 15° est supérieur ou égal à 70%, mieux supérieur ou égal à 90%, et encore mieux supérieur ou égal à 95%.

Dans un mode de réalisation préféré de l'invention, le filtre que comporte la lentille est un filtre interférentiel. On entendra par là que le filtre comprend au moins une couche formée sur l'une des faces principales de la lentille ophtalmique munie du filtre interférentiel, cette couche présentant un indice de réfraction différent d'au moins 0,1 unité de l'indice de réfraction du substrat. Les propriétés optiques d'un tel filtre, comme par exemple la réflectivité, résultent des interférences provenant des réflexions multiples aux interfaces air / couche et substrat / couche.

Une couche du filtre est définie comme ayant une épaisseur déposée supérieure ou égale à 1 nm. Ainsi, toute couche ayant une épaisseur inférieure à 1 nm ne sera pas comptabilisée dans le nombre de couches du filtre. Une éventuelle sous-couche disposée entre le filtre et le substrat n'est pas non plus comptabilisée dans le nombre de couches du filtre interférentiel.

Sauf indication contraire, toutes les épaisseurs de couches divulguées dans la présente demande sont des épaisseurs physiques, et non des épaisseurs optiques.

Lorsque le filtre interférentiel de l'invention comprend au moins deux couches, il comprend alors un empilement d'au moins une couche de haut indice de réfraction, ou « couche haut indice » désignée couche HI, et d'au moins une couche de bas indice de réfraction, ou « couche bas indice » désignée couche BI.

Dans un mode de réalisation préféré, le filtre interférentiel comporte moins de 11 couches, de préférence un nombre de couches allant de 2 à 10 couches, mieux de 4 à 9 couches, de façon optimale de 4 à 7 couches. Il n'est pas nécessaire que les couches HI et BI soient alternées dans l'empilement du filtre interférentiel, bien qu'elles puissent l'être selon un mode de réalisation de l'invention. Deux couches HI (ou plus) peuvent être déposées l'une sur l'autre, tout comme deux couches BI (ou plus) peuvent être déposées l'une sur l'autre.

Dans la présente demande, une couche du filtre interférentiel est dite couche de haut indice de réfraction » lorsque son indice de réfraction est supérieur à 1,60, de préférence supérieur ou égal à 1,65, de préférence encore supérieur ou égal à 1,70, mieux supérieur ou égal à 1,80 et encore mieux supérieur ou égal à 1,90. De même, une couche du filtre interférentiel est dite couche de bas indice de réfraction lorsque son indice de réfraction est inférieur à 1,50, de préférence inférieur ou égal à 1,48, mieux inférieur ou égal à 1,47.

Sauf indication contraire, les indices de réfraction auxquels il est fait référence dans la présente demande sont exprimés à une température de 25°C et pour une longueur d'onde de référence égale à 550 nm.

La couche HI est une couche de haut indice de réfraction classique, bien connue dans la technique. Elle comprend généralement un ou plusieurs oxydes minéraux tels que, sans limitation : la zircone ($ZrO_2$), l'oxyde de titane ($TiO_2$), l'alumine ($Al_2O_3$), le pentoxyde de tantale ($Ta_2O_5$), l'oxyde de néodyme ($Nd_2O_5$), l'oxyde de praséodyme ($Pr_2O_3$), le titanate de praséodyme ($PrTiO_3$), l'oxyde de lanthane ($La_2O_3$), le pentoxyde de niobium ($Nb_2O_5$), ou l'oxyde d'yttrium ($Y_2O_3$). Éventuellement, les couches HI peuvent contenir également de la silice ou d'autres matériaux de bas indice de réfraction, pourvu que leur indice de réfraction soit supérieur à 1,60 comme indiqué ci-dessus. Les matériaux préférés sont $TiO_2$, $PrTiO_3$, $ZrO_2$, $Al_2O_3$, $Y_2O_3$ et leurs mélanges.

La couche BI est également couche de bas indice de réfraction classique bien connue et peut comprendre, sans limitation : de la silice ($SiO_2$), ou bien un mélange de silice et d'alumine, en particulier de la silice dopée avec de l'alumine, cette dernière contribuant à augmenter la résistance thermique du filtre interférentiel. La couche BI est de préférence une couche comprenant au moins 80% en masse de silice, mieux au moins 90 % en masse de silice, par rapport à la masse totale de la couche BI, et encore mieux consiste en une couche de silice.

Éventuellement, les couches bas indice peuvent contenir également des matériaux de haut indice de réfraction, pourvu que l'indice de réfraction de la couche résultante soit inférieur à 1,50.

Lorsqu'une couche BI comprenant un mélange de $SiO_2$ et d'$Al_2O_3$ est utilisée, elle comprend préférentiellement de 1 à 10%, mieux de 1 à 8% et encore mieux de 1 à 5% en masse d'$Al_2O_3$ par rapport à la masse totale de silice et d'alumine dans cette couche.

Par exemple, des couches de $SiO_2$ dopée avec 4% ou moins d'$Al_2O_3$ en masse, ou une couche de $SiO_2$ dopée avec 8% d'$Al_2O_3$ peuvent être employées. Des mélanges $SiO_2$/ $Al_2O_3$ disponibles dans le commerce peuvent être utilisés, tels que le LIMA® commercialisé par UMICORE MATERIALS AG (indice de réfraction compris entre 1,48 et 1,50), ou la substance L5® commercialisée par MERCK KGaA (indice de réfraction égale à 1,48 pour une longueur d'onde de 500 nm).

La couche externe du filtre interférentiel est généralement une couche bas indice, typiquement à base de silice, comprenant de préférence au moins 80% en masse de silice, mieux au moins 90% en masse de silice (par exemple une couche de silice dopée avec de l'alumine), par rapport à la masse totale de cette couche externe, et encore mieux consiste en une couche externe de silice.

Dans un mode de réalisation préféré, le filtre présente une épaisseur totale inférieure ou égale à 700 nanomètres, mieux inférieure ou égale à 600 nm. L'épaisseur totale du filtre est généralement supérieure à 200 nm, de préférence supérieure à 250 nm.

Dans les modes de réalisation particuliers de l'invention où le filtre est un filtre interférentiel comprenant 8 ou 9 couches, l'épaisseur totale de l'empilement est de préférence comprise entre 450 nm et 600 nm.

Dans les modes de réalisation particuliers de l'invention où le filtre est un filtre interférentiel comprenant 6 ou 7 couches, l'épaisseur totale de l'empilement est de préférence inférieure à 500 nm, mieux elle est comprise entre 300 nm et 500 nm.

Dans les modes de réalisation particuliers de l'invention où le filtre est un filtre interférentiel comprenant 4 ou 5 couches, l'épaisseur totale de l'empilement est de préférence inférieure à 300 nm, mieux elle est comprise entre 200 nm et 300 nm.

Généralement, les couches HI ont une épaisseur physique variant de 10 nm à 100 nm, mieux inférieure ou égale à 80 nm, et encore mieux inférieure ou égale à 70 nm, et les couches BI ont une épaisseur physique variant de 10 nm à 150 nm, mieux inférieure ou égale à 135 nm, et encore mieux inférieure ou égale à 120 nm.

La lentille ophtalmique de l'invention peut également être rendue antistatique, c'est-à-dire ne pas retenir et/ou développer une charge électrostatique appréciable, grâce à l'incorporation d'au moins une couche électriquement conductrice dans le filtre.

De préférence, il s'agit d'une couche supplémentaire d'un oxyde conducteur tel que l'oxyde d'indium, l'oxyde d'étain, l'ITO (Indium Tin Oxide). Cette couche présente une épaisseur généralement inférieure à 20 nm, préférentiellement entre 5 nm et 15 nm.

Elle est préférentiellement adjacente à une couche de haut indice telle qu'une couche d'oxyde de zirconium.

Préférentiellement, cette couche conductrice est disposée sous la dernière couche bas indice du filtre (c'est-à-dire la couche la plus proche de l'air), généralement à base de silice,

Selon un mode de réalisation de l'invention, le filtre est déposé sur une sous-couche. Il est considéré ici que cette sous-couche du filtre ne fait pas partie du filtre.

Par sous-couche du filtre, ou couche d'adhésion, on entend un revêtement d'épaisseur relativement importante, utilisé dans le but d'améliorer les propriétés mécaniques telles que la résistance à l'abrasion et/ou à la rayure du filtre et/ou de promouvoir son adhésion au substrat ou au revêtement sous-jacent.

Compte tenu de son épaisseur relativement importante, la sous-couche ne participe généralement pas à l'activité optique de filtrage du filtre, en particulier dans le cas où elle possède un indice de réfraction proche de celui du revêtement sous-jacent (qui est généralement le revêtement anti-abrasion et/ou anti-rayures) ou de celui du substrat de la lentille ophtalmique, lorsque la sous-couche est directement déposée sur le substrat de la lentille ophtalmique.

La sous-couche doit avoir une épaisseur suffisante pour promouvoir la résistance à l'abrasion du filtre, mais de préférence pas trop importante pour ne pas provoquer une absorption lumineuse qui, selon la nature de la sous-couche, pourrait réduire significativement le facteur de transmission visuelle $T_v$ tel que défini dans la norme ISO 13666 :1998, et mesuré conformément à la norme ISO 8980-3.

L'épaisseur de cette sous-couche est généralement inférieure à 300 nm, mieux inférieur à 200 nm, et est généralement supérieure à 90 nm, mieux supérieure à 100 nm.

La sous-couche comprend de préférence une couche à base de $SiO_2$, comprenant de préférence au moins 80% en masse de silice, mieux au moins 90% en masse de silice, par rapport à la masse totale de la sous-couche, et encore mieux consiste en une sous-couche de silice. L'épaisseur de cette sous-couche à base de silice est généralement inférieure à 300 nm, mieux inférieure à 200 nm, et est généralement supérieure à 90 nm, mieux supérieure à 100 nm.

Selon un autre mode de réalisation, cette sous-couche à base de $SiO_2$ est une sous-couche de silice dopée avec de l'alumine, dans des proportions telles que définies ci-dessus, de préférence consiste en une couche de silice dopée avec de l'alumine.

Selon un mode de réalisation particulier, la sous-couche consiste en une couche de $SiO_2$.

Il est préférable d'utiliser une sous-couche de type monocouche. Toutefois, la sous-couche peut être laminée (multicouches), en particulier lorsque la sous-couche et le revêtement sous-jacent (ou le substrat, si la sous-couche est déposée directement sur le substrat) présentent une différence d'indice de réfraction non négligeable. C'est notamment le cas lorsque le revêtement sous-jacent, qui est généralement un revêtement anti-abrasion et/ou anti-rayure, ou le substrat, possèdent un indice de réfraction élevé, terme par lequel on entend un indice de réfraction supérieur ou égal à 1,55, de préférence supérieur ou égal à 1,57.

Dans ce cas, la sous-couche peut comporter, outre une couche d'épaisseur comprise entre 90 nm et 300 nm, dite couche principale, de préférence au plus trois autres couches, mieux au plus deux autres couches, intercalées entre le substrat éventuellement revêtu et cette couche d'épaisseur comprise entre 90 nm et 300 nm, qui est généralement une couche à base de silice. Ces couches additionnelles sont de préférence de fines couches, dont la fonction est de limiter les réflexions multiples à l'interface sous-couche / revêtement sous-jacent ou à l'interface sous-couche / substrat, selon le cas.

Une sous-couche multicouche comprend préférentiellement, outre la couche principale, une couche d'indice de réfraction élevé et d'épaisseur inférieure ou égale à 80 nm, mieux inférieure ou égale à 50 nm et mieux encore inférieure ou égale à 30 nm. Cette couche d'indice de réfraction élevé est directement en contact avec le substrat d'indice de réfraction élevé ou le revêtement sous-jacent d'indice de réfraction élevé, selon le cas. Bien entendu, ce mode de réalisation peut être utilisé même si le substrat (ou le revêtement sous-jacent) possède un indice de réfraction inférieur à 1,55.

En alternative, la sous-couche comprend, outre la couche principale et la couche d'indice de réfraction élevé précitée, une couche de matériau d'indice de réfraction inférieur ou égal à 1,55, de préférence inférieur ou égal à 1,52, mieux inférieur ou égal à 1,50 à base de $SiO_2$ (c'est-à-dire comprenant de préférence au moins 80% en masse de silice) d'épaisseur inférieure ou égale à 80 nm, mieux inférieure ou égale à 50 nm et mieux encore inférieure ou égale à 30 nm, sur laquelle est déposée ladite couche d'indice de réfraction élevé. Typiquement, dans ce cas, la sous-couche comprend, déposées dans cet ordre sur le substrat éventuellement revêtu, une couche de 25 nm de $SiO_2$, une couche de 10 nm de $ZrO_2$ ou de $Ta_2O_5$ et la couche principale de la sous-couche.

Le filtre et l'éventuelle sous-couche sont préférentiellement déposées par dépôt sous vide selon l'une des techniques suivantes : i) par évaporation, éventuellement assistée par faisceau ionique ; ii) par pulvérisation par faisceau d'ions; iii) par pulvérisation cathodique ; iv) par dépôt chimique en phase vapeur assisté par plasma. Ces différentes techniques sont décrites dans les ouvrages *"Thin Film Processes"* et *"Thin Film Processes II"* (Vossen & Kern Éditeurs, Academic Press, 1978 et 1991 respectivement). Une technique particulièrement recommandée est la technique d'évaporation sous vide.

De préférence, lorsque le filtre est un filtre interférentiel, le dépôt de chacune des couches de l'empilement du filtre et de l'éventuelle sous-couche est réalisé par évaporation sous vide.

Dans un mode de réalisation particulier de l'invention, la lentille ophtalmique présente un facteur moyen de réflexion lumineux $R_v$ sur la face principale de la lentille ophtalmique comportant le filtre qui est inférieur ou égal à 2,5%. De préférence, ce facteur moyen de réflexion lumineux $R_v$ est inférieur ou égal à 2%, mieux encore inférieur ou égal à 1,5%. Dans un mode particulièrement préféré, le facteur moyen de réflexion lumineux $R_v$ est inférieur ou égal à 0,7%, mieux inférieur ou égal à 0,6%.

Dans un mode de réalisation préféré, la lentille ophtalmique présente un facteur moyen de réflexion lumineux $R_v$ sur chacune des faces principales de la lentille ophtalmique qui est inférieur ou égal à 2,5%. Mieux, ce facteur moyen de réflexion lumineux $R_v$ est inférieur ou égal à 0,7%.

Selon un mode de réalisation préféré de l'invention, la face principale revêtue par le filtre selon l'invention est la face principale avant de la lentille ophtalmique de l'invention et la face principale arrière est revêtue d'un revêtement antireflet conventionnel ou bien de préférence d'un revêtement antireflet efficace dans l'UV, c'est-à-dire réfléchissant peu les UV, tel que ceux décrits par exemple dans le document PCT/EP2011/072386.

Le facteur moyen de réflexion dans l'UV $R_{UV}$ sur la face principale arrière de la lentille ophtalmique, pour des longueurs d'onde comprises entre 280 nm et 380 nm, pondéré par la fonction $W(\lambda)$ définie dans la norme ISO 13666:1998, est inférieur ou égal à 7%, mieux inférieur ou égal à 6%, et mieux encore inférieur ou égal à 5%, pour un angle d'incidence de 30° et pour un angle d'incidence de 45°. Le facteur moyen de réflexion dans l'UV Ruv est défini par la relation :

$$R_{UV} = \frac{\int\limits_{280}^{380} W(\lambda).R(\lambda).d\lambda}{\int\limits_{280}^{380} W(\lambda).d\lambda}$$

où R($\lambda$) désigne le réflectivité spectrale sur la face principale arrière de la lentille ophtalmique à la longueur d'onde considérée, et W($\lambda$) désigne une fonction de pondération égale au produit de l'éclairement énergétique spectral solaire Es($\lambda$) par la fonction spectrale relative d'efficacité S($\lambda$).

La fonction spectrale W($\lambda$), qui permet de calculer les facteurs de transmission des rayonnements UV est définie dans la norme ISO 13666:1998.

Le revêtement antireflet efficace dans l'UV comprend de préférence un empilement d'au moins une couche de haut indice de réfraction et d'au moins une couche de bas indice de réfraction.

Dans un autre mode de réalisation de l'invention, les deux faces principales avant et arrière comportent chacune un filtre contre la lumière bleue phototoxique. Les deux filtres ainsi formés, l'un sur la face principale avant et l'autre sur la face principale arrière, peuvent alors être identiques ou différents.

Le filtre selon l'invention peut être déposé directement sur un substrat nu. Dans certaines applications, il est préférable que la face principale de la lentille ophtalmique comportant le filtre soit revêtue d'un ou plusieurs revêtements fonctionnels préalablement à la formation du filtre sur cette face principale. Ces revêtements fonctionnels, classiquement utilisés en optique, peuvent être, sans limitation : une couche de primaire anti-choc, un revêtement anti-abrasion et/ou anti-rayures, un revêtement polariseur, un revêtement coloré.

Généralement, la face principale avant et/ou arrière du substrat sur laquelle sera formé un filtre est revêtue d'une couche de primaire anti-choc, d'un revêtement anti-abrasion et/ou anti-rayures.

Le filtre est de préférence déposé sur un revêtement anti-abrasion et/ou anti-rayures. Le revêtement anti-abrasion et/ou anti-rayures peut être toute couche classiquement utilisée comme revêtement anti-abrasion et/ou anti-rayure dans le domaine des lentilles ophtalmiques. De tels revêtements sont décrits, entre autres, dans le document EP 0614957.

La lentille ophtalmique selon l'invention peut également comporter des revêtements formés sur le filtre et capables de modifier ses propriétés de surface, tels que des revêtements hydrophobes et/ou oléophobes (« *top coat* » anti-salissure) et/ou des revêtements anti-buée. De tels revêtements sont décrits, entre autres, dans le document US 7678464. Ces revêtements sont de préférence déposés sur la couche externe du filtre. Leur épaisseur est en général inférieure ou égale à 10 nm, de préférence de 1 nm à 10 nm, mieux de 1 nm à 5 nm.

Typiquement, une lentille ophtalmique selon l'invention comprend un substrat successivement revêtu sur sa face principale avant d'une couche de primaire anti-choc, d'une couche anti-abrasion et/ou anti-rayures, d'un filtre selon l'invention, et d'un revêtement hydrophobe et/ou oléophobe.

La lentille ophtalmique selon l'invention est de préférence une lentille ophtalmique pour lunettes, ou une ébauche de lentille ophtalmique. L'invention concerne ainsi également une paire de lunettes comportant au moins une telle lentille ophtalmique.

La lentille ophtalmique peut être une lentille polarisée, ou une lentille solaire, teintée, avec ou sans correction.

La face principale arrière du substrat de l'article d'optique peut être revêtue successivement d'une couche de primaire anti-choc, d'une couche anti-abrasion et/ou anti-rayures, d'un revêtement anti-reflet qui peut être, ou non, un revêtement antireflet anti-UV, et d'un revêtement hydrophobe et/ou oléophobe.

Elle est particulièrement avantageuse pour protéger de la phototoxicité de la lumière bleue l'œil d'un porteur souffrant d'une détérioration d'un œil, en particulier due à un processus dégénératif tel que la dégénérescence maculaire liée à l'âge.

Une lentille ophtalmique telle que décrite ci-dessus présente également l'avantage de conférer un meilleur contraste visuel au porteur.

Les exemples suivants illustrent l'invention de façon plus détaillée mais non limitative.

EXEMPLES

1. Procédures générales et modes opératoires

Les filtres selon l'invention sont déposés sur des verres ORMA® revêtus d'un revêtement anti-abrasion tel que décrit dans l'exemple 3 du brevet EP614957.

Le dispositif d'évaporation et les conditions de dépôt des couches de $SiO_2$ et de $ZrO_2$ (vitesse d'évaporation,

pression) sont telles que décrites dans la demande de brevet WO 2008107325.

2. Calcul des courbes

Les courbes de réflectivité spectrales des filtres selon l'invention ont été modélisées à partir du logiciel *Essential Mac Leod* (version 9.4) de Thin Film Center.
Les caractéristiques des filtres et leurs propriétés figurent au point 3 ci-dessous.
Les lentilles ophtalmiques équipées des filtres des exemples 1 et 2 ont effectivement été réalisées et les courbes de réflectivités spectrales mesurées.
Il a été vérifié que les courbes obtenues correspondaient à celles modélisées.

3. Empilements des filtres et propriétés. Courbes de réflectivité spectrale. Résultats

Les caractéristiques structurelles et les performances optiques des lentilles ophtalmiques obtenues selon les exemples 1 à 3 sont détaillées ci-dessous (voir page suivante).
Les courbes de réflectivité spectrale à un angle d'incidence sur la face principale avant de 15° et pour des longueurs d'onde allant de 280 nm à 780 nm des exemples 1 à 3 ci-dessous sont représentées sur les figures 1 à 3
Les valeurs des facteurs moyens de réflexion sont celles de la face principale avant. Les facteurs $R_{m,B}$ et $R_v$ sont indiqués pour un angle d'incidence de 15°.
Dans le tableau suivant, le paramètre $\Delta$ **spectral @ 15°** est défini par la relation: $\Delta$ **spectral @ 15°**=$[R_{15°}(435nm)-R_{15°}(480nm)]/R_{15°}(435nm)$ où $R_{15°}(435$ nm) et $R_{15°}(480$ nm) représentent respectivement la réflectivité de la face principale avant à 435 nm et à 480 nm, pour un angle d'incidence de 15° sur la face principale avant.
On constate que les lentilles ophtalmiques selon l'invention possèdent de très bonnes propriétés de réflexion de la lumière bleue phototoxique ($R_{m,B} > 10\%$), sans que cela ne nuise aux performances anti-reflet dans le domaine visible ($R_v < 2,5\%$ pour un angle d'incidence de 15°).
Les lentilles ophtalmiques obtenues selon les exemples 1 à 3 présentent en outre d'excellentes propriétés de transparence et une bonne neutralité colorimétrique, une bonne résistance à l'abrasion et aux rayures, et une bonne résistance à un traitement au trempé dans l'eau chaude suivi d'une sollicitation mécanique de surface. L'adhérence des revêtements au substrat est également très satisfaisante.

| Ex. 1 : filtre large à 4 couches sur la face principale avant | | Ex. 2 : filtre large à 6 couches sur la face principale avant | | Ex. 3 : filtre large à 8 couches sur la face principale avant | |
|---|---|---|---|---|---|
| | | | | | |
| Substrat + hard coat | | Substrat + hard coat | | Substrat + hard coat | |
| ZrO2 | 34 nm | ZrO2 | 44 nm | ZrO2 | 47 nm |
| SiO2 | 35 nm | SiO2 | 45 nm | SiO2 | 50 nm |
| ZrO2 | 73 nm | Zr02 | 68 nm | ZrO2 | 54 nm |
| SiO2 | 110 nm | SiO2 | 32 nm | SiO2 | 70 nm |
| Air | | ZrO2 | 66 nm | ZrO2 | 45 nm |
| | | SiO2 | 124 nm | SiO2 | 62 nm |
| | | Air | | ZrO2 | 53 nm |
| | | | | SiO2 | 134 nm |
| | | | | Air | |

| Épaisseur totale | 252 nm | Épaisseur totale | 379 nm | Épaisseur totale | 515 nm |
|---|---|---|---|---|---|
| Rm,B @ 15° (420 - 450nm) | 11,8% | Rm,B @ 15° (420 - 450nm) | 30,6% | Rm,B @ 15° (420 - 450nm) | 51,5% |
| Max. Réflectivité | 359 nm | Max. Réflectivité | 379 nm | Max. Réflectivité | 384 nm |

(suite)

| Largeur à mihauteur | 98 nm | | Largeur à mihauteur | 100 nm | | Largeur à mi-hauteur | 105 nm |
|---|---|---|---|---|---|---|---|
| $\Delta(\theta=15°,\theta'=45°)$@ 435 nm | 0,72 | | $\Delta(\theta=15°,\theta'=45°)$@ 435 nm | 0,75 | | $\Delta(\theta=15°,\theta'=45°)$@ 435 nm | 0,80 |
| Rm @ 15° (465 - 495 nm) | 2,3% | | Rm @ 15° (465 - 495 nm) | 4,5% | | Rm @ 15° (465 - 495 nm) | 7,3% |
| $\Delta$ spectral @ 15°435 nm/480 nm | 0,85 | | $\Delta$ spectral @ 15° 435 nm/480 nm | 0,92 | | $\Delta$ spectral @ 15° 435 nm/480 nm | 0,95 |
| Rv @ 15° (380 - 780 nm) | 0,5% | | Rv @ 15° (380 - 780 nm) | 1,9% | | Rv @ 15° (380 - 780 nm) | 2,0% |

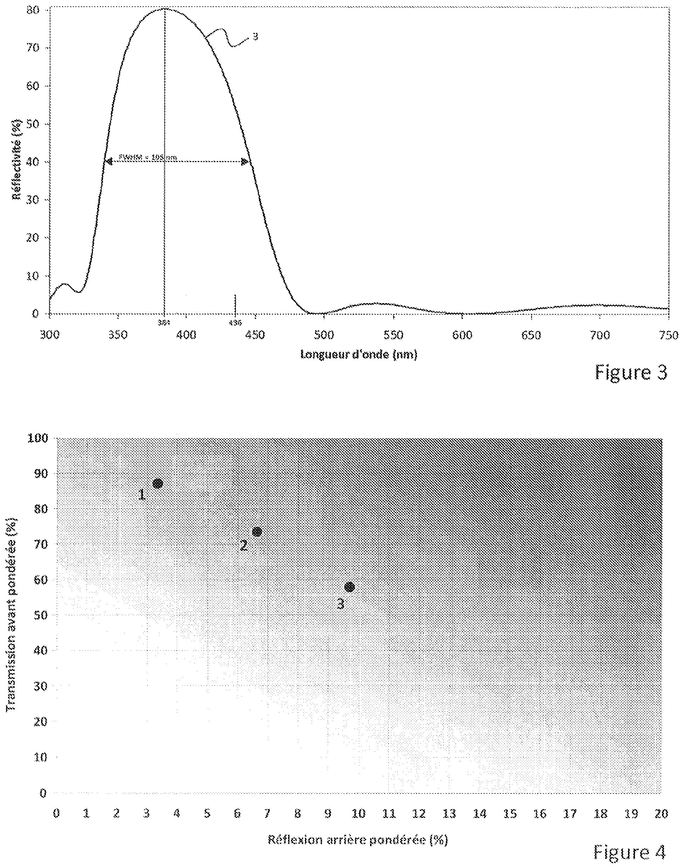

Par ailleurs, on comprendra à la lumière de la figure 4 l'efficacité des exemples 1 à 3 de lentilles ophtalmiques selon l'invention.

La réflexion arrière $BR(\lambda)$ et la transmission $T(\lambda)$ de l'ensemble du système optique (avec le filtre bleu correspondant aux exemples 1, 2 et 3, en face avant d'une lentille de type biplan en verre ORMA®, portant un antireflet Crizal Forte® UV en face arrière ($R_v$ = 0,59%, Ruv = 3,1% pour une incidence de 45°), ont été déterminées à l'aide du logiciel *Essential Mac Leod* pour chacun des filtres étudiés.

Le calcul tient compte de l'ensemble des multiples réflexions se produisant au sein de la lentille ophtalmique.

Pour évaluer le risque associé à la lumière bleue, ces courbes de transmission et de réflexion sont pondérées à l'aide de la fonction spectrale $W_B(\lambda)$ issue de la norme internationale ISO 8980-3. Cette fonction résulte du produit de la fonction de risque de la lumière bleue (« *blue-light hazord function* » en anglais) $B(\lambda)$ et de la fonction de distribution spectrale du soleil (« *spectral distribution of solar radiation* » en anglais) $E_S(\lambda)$ intégrées sur la gamme de longueurs d'onde allant de 380 nm à 500 nm.

*Fonctions spectrales pour le calcul des valeurs de transmission ou réflexion de la lumière dans la bande 380-500 nm :*

**Tableau 1** : Données numériques permettant de calculer la fonction de pondération $W_B(\lambda)$.

| Longueur d'onde $\lambda$ (nm) | Irradiation solaire spectrale Es $(\lambda)$ (mW/m².nm) | Fonction de risque de la lumière bleue $B(\lambda)$ | Fonction de pondération $W_B(\lambda)$ = Es$(\lambda)$.$B(\lambda)$ |
|---|---|---|---|
| 380 | 336 | 0.006 | 2 |
| 385 | 365 | 0,012 | 4 |
| 390 | 397 | 0,025 | 10 |
| 395 | 432 | 0,05 | 22 |
| 400 | 470 | 0,10 | 47 |
| 405 | 562 | 0,20 | 112 |
| 410 | 672 | 0,40 | 269 |
| 415 | 705 | 0,80 | 564 |
| 420 | 733 | 0,90 | 660 |
| 425 | 760 | 0,95 | 722 |
| 430 | 787 | 0,98 | 771 |
| 435 | 849 | 1,00 | 849 |
| 440 | 911 | 1,00 | 911 |
| 445 | 959 | 0,97 | 930 |
| 450 | 1006 | 0,94 | 946 |
| 455 | 1037 | 0,90 | 933 |
| 460 | 1080 | 0,80 | 864 |

(suite)

| Longueur d'onde $\lambda$ (nm) | Irradiation solaire spectrale Es $(\lambda)$ (mW/m².nm) | Fonction de risque de la lumière bleue B($\lambda$) | Fonction de pondération $W_B(\lambda) = Es(\lambda).B(\lambda)$ |
|---|---|---|---|
| 465 | 1109 | 0,70 | 776 |
| 470 | 1138 | 0,62 | 706 |
| 475 | 1161 | 0,55 | 639 |
| 480 | 1183 | 0,45 | 532 |
| 485 | 1197 | 0,40 | 479 |
| 490 | 1210 | 0,22 | 266 |
| 495 | 1213 | 0,16 | 194 |
| 500 | 1215 | 0,10 | 122 |

Sur la figure 4, sont représentés :

- en abscisses : la valeur de la réflexion arrière pondérée par la fonction de pondération du risque de la lumière bleue, pour un angle d'incidence sur la face principale arrière de 45°.

$$ BR_B = \frac{\int_{380}^{500} W_B(\lambda).BR(\lambda).d\lambda}{\int_{380}^{500} W_B(\lambda).d\lambda} $$

où $BR(\lambda)$ est le facteur spectral de réflexion arrière du verre (« back reflectance » en anglais), et

- en ordonnées : la valeur de la transmission pondérée par la fonction de pondération du risque de la lumière bleue. Cette valeur de transmission représente la part (en %) de lumière directe transmise dans la bande bleu-violet (de 380 nm à 500 nm) par cette lentille ophtalmique pour un angle d'incidence sur la face principale avant de 0°.

$$ T_B = \frac{\int_{380}^{500} W_B(\lambda).T(\lambda).d\lambda}{\int_{380}^{500} W_B(\lambda).d\lambda} $$

où $T(\lambda)$ est le facteur spectral de transmission du verre.

La grandeur $W_B(\lambda)$ représente la fonction de pondération qui est égale au produit de l'irradiation solaire spectrale $Es(\lambda)$ et de la fonction de risque de la lumière bleue $B(\lambda)$ (voir tableau 1).

On constate sur la figure 4 que les exemples 1 à 3 de lentilles ophtalmiques selon l'invention présentent non seulement une faible transmission, mais aussi une réflexion arrière faible.

Ainsi, les lentilles ophtalmiques des exemples de l'invention permettent de prévenir un processus dégénératif de l'œil d'un porteur dû à la phototoxicité de la lumière bleue, tel que la dégénérescence maculaire liée à l'âge,

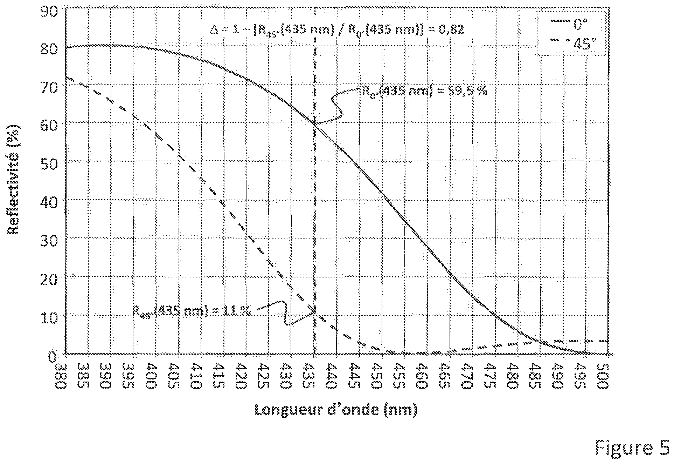

La figure 5 représente les courbes de réflectivité spectrale entre 380 nm et 500 nm de la lentille ophtalmique de l'exemple 3 pour des angles d'incidence sur la face principale avant de 0° et de 45°.

On remarque sur cette figure que la courbe de réflectivité spectrale à 45° est décalée vers les courtes longueurs d'onde (i.e. vers le bleu profond et l'UV) par rapport à la courbe de réflectivité spectrale à 0°. Ceci est l'illustration de la forte sélectivité angulaire du filtre large de l'exemple 3.

Ce décalage entraîne alors que la valeur de la réflectivité spectrale à 435nm pour un angle d'incidence de 45°, notée ici $R_{45°}$(435 nm) est faible, ici égale à 11%, et bien inférieure à la valeur de la réflectivité spectrale à 435nm pour un angle d'incidence de 0°, notée ici $P_{0°}$(435 nm), égale à 59,5%.

On comprend donc que la valeur du paramètre Δ(θ,θ') est ici élevée, égale à 0,82. Ceci est vrai pour toutes les lentilles ophtalmiques, qui sont munies d'au moins un filtre large selon l'invention.

**Revendications**

1. Lentille ophtalmique présentant une face principale avant et une face principale arrière comprenant :

   - un moyen de coupure de la lumière ultra-violette (UV) arrivant sur la face principale avant de la lentille ophtalmique, ledit moyen de coupure étant constitué par un substrat de la lentille ophtalmique ;
   - un revêtement antireflet, sur la face principale arrière de la lentille ophtalmique, ayant un facteur moyen de réflexion dans l'UV pondéré par la fonction $W(\lambda)$ définie dans la norme ISO 13666:1998 inférieur ou égal à 7% ; et
   - au moins un filtre bloquant au moins en partie la lumière bleue dans le domaine de longueurs d'onde allant de 420 à 450 nanomètres, ledit filtre étant formé sur une face principale de la lentille et **caractérisé en ce qu'**il confère à la face principale comportant ledit filtre, la propriété suivante :

   o pour un angle d'incidence θ compris entre 0° et 15° et pour un angle d'incidence θ' compris entre 30° et 45°, un paramètre Δ(θ, θ') défini par la relation $\Delta(\theta, \theta') = 1 - [R_{\theta'}(435\,nm) / R_{\theta}(435\,nm)]$ tel que ce paramètre Δ(θ, θ') soit supérieur ou égal à 0,5, de préférence supérieur ou égal à 0,6, où :

   ▪ $R_{\theta}(435\,nm)$ représente la valeur de la réflectivité de la face principale comportant ledit filtre à la longueur d'onde de 435 nanomètres pour l'angle d'incidence θ, et
   ▪ $R_{\theta'}(435\,nm)$ représente la valeur de la réflectivité de la face principale comportant ledit filtre à la longueur d'onde de 435 nanomètres pour l'angle d'incidence θ'.

2. Lentille ophtalmique selon la revendication 1, dans laquelle ledit filtre est un filtre par réflexion permettant une excellente transmission dans la gamme de longueurs d'onde allant de 465 à 495 nm.

3. Lentille ophtalmique selon l'une des revendications 1 et 2, dans laquelle le moyen de coupure de la lumière UV arrivant sur la face principale avant coupe 90% ou plus de la lumière UV.

4. Lentille ophtalmique selon l'une des revendications 1 à 3, dans laquelle ledit filtre bloquant au moins en partie la lumière bleue est formé sur la face principale avant de ladite lentille ophtalmique.

5. Lentille ophtalmique selon l'une quelconque des revendications précédentes, dans laquelle le filtre bloquant la lumière bleue bloque de 5 à 50% de la lumière bleue dans le domaine de longueurs d'onde de 420 à 450 nanomètres.

6. Lentille ophtalmique selon l'une quelconque des revendications précédentes, dans laquelle ledit filtre confère à la face principale de la lentille ophtalmique comprenant le filtre la propriété de présenter, pour un angle d'incidence sur cette face principale compris entre 0° et 15°, un facteur moyen de réflexion dans le bleu $R_{m,B}$ qui est supérieur ou égal à 5%.

7. Lentille ophtalmique selon l'une des revendications précédentes, dans laquelle l'angle d'incidence θ = 15° et l'angle d'incidence θ' = 45°.

8. Lentille ophtalmique selon l'une des revendications précédentes, dans laquelle le filtre bloquant la lumière bleue confère en outre à la face principale comportant ledit filtre la propriété suivante :

   - une courbe de réflectivité spectrale pour un angle d'incidence compris entre 0° et 15° ayant :

   ◦ un maximum de réflectivité à une longueur d'onde inférieure à 435 nanomètres, et
   ◦ une largeur à mi-hauteur (FWHM) supérieure ou égale à 70 nm, de préférence supérieure ou égale à 75 nm et mieux supérieure ou égale à 80 nm et inférieure à 150 nm, de préférence inférieure ou égale à 120 nm et mieux inférieure ou égale à 110 nm.

9. Lentille ophtalmique selon la revendication 8, dans laquelle ledit maximum de réflectivité est à une longueur d'onde inférieure ou égale à 400 nm, mieux inférieure ou égale à 390 nm.

10. Lentille ophtalmique selon la revendication 8 ou 9, dans laquelle ledit maximum de réflectivité est à une longueur d'onde supérieure ou égale à 350 nm.

11. Lentille ophtalmique selon l'une quelconque des revendications précédentes, dans laquelle le facteur moyen de réflexion lumineux ($R_v$) sur la face principale de la lentille ophtalmique comportant ledit filtre est inférieur ou égal à 2,5%.

12. Lentille ophtalmique selon l'une quelconque des revendications précédentes, dans laquelle le facteur moyen de réflexion lumineux ($R_v$) sur chacune des faces principales de la lentille ophtalmique est inférieur ou égal à 2,5%, préférentiellement inférieur ou égal à 1,5%.

13. Lentille ophtalmique selon l'une quelconque des revendications précédentes, dans laquelle le facteur moyen pondéré de réflexion dans l'UV de l'antireflet sur la face principale arrière est inférieur ou égal à 6%, de préférence inférieur ou égal à 5%, mieux inférieur ou égal à 4,5% et idéalement inférieur ou égal à 3,5%.

14. Lentille ophtalmique selon l'une quelconque des revendications précédentes, dans laquelle le filtre est un filtre interférentiel.

15. Lentille ophtalmique selon l'une quelconque des revendications précédentes, dans laquelle le ratio $\Delta$ spectral @ 15°=[$R_{15°}$(435nm)-$R_{15°}$(480nm)]/$R_{15°}$(435nm) où $R_{15°}$(435nm) et $R_{15°}$(480 nm) représentent respectivement la réflectivité de la face principale de la lentille ophtalmique comportant ledit filtre à une longueur d'onde de 435 nm et à une longueur d'onde de 480 nm, pour un angle d'incidence sur cette face principale de 15°, est supérieur ou égal à 0,8, mieux supérieur ou égal à 0,85 et mieux encore supérieur ou égal à 0,9.

16. Verre de lunette constitué par une lentille ophtalmique selon l'une quelconque des revendications précédentes, **caractérisé en ce qu'**il est choisi parmi les verres blancs, les verres traités, les verres solaires et les verres photochromiques.

**Patentansprüche**

1. Ophthalmische Linse, die eine vordere Hauptseite und eine hintere Hauptseite aufweist und Folgendes umfasst:

   - ein Mittel zum Sperren des ultravioletten (UV) Lichts, das auf die vordere Hauptseite der ophthalmischen Linse trifft, wobei das Mittel zum Sperren durch ein Substrat der ophthalmischen Linse gebildet wird;
   - eine Antireflex-Beschichtung auf der hinteren Hauptseite der ophthalmischen Linse, die einen mittleren Reflexionsfaktor im UV-Bereich, der durch die in der Norm ISO 13666:1998 definierte Funktion $W(\lambda)$ gewichtet wird, besitzt, der kleiner oder gleich 7 % ist; und
   - mindestens einen Filter, der das blaue Licht im Wellenlängenbereich von 420 bis 450 Nanometer zumindest teilweise blockiert, wobei der Filter auf einer Hauptseite der Linse gebildet ist und **dadurch gekennzeichnet ist, dass** er der diesen Filter beinhaltenden Hauptseite die folgende Eigenschaft verleiht:

     - bei einem Einfallswinkel $\theta$ zwischen 0° und 15° und bei einem Einfallswinkel $\theta'$ zwischen 30° und 45° einen Parameter $\Delta(\theta, \theta')$, der durch die Gleichung $\Delta(\theta, \theta') = 1 - [R_{\theta'}(435\,nm)/R_{\theta}(435\,nm)]$ definiert wird, so dass dieser Parameter $\Delta(\theta, \theta')$ größer oder gleich 0,5, bevorzugt größer oder gleich 0,6 ist, wobei:

       - $R_{\theta}(435\,nm)$ den Wert der Reflektivität der den Filter beinhaltenden Hauptseite bei der Wellenlänge von 435 Nanometern beim Einfallswinkel $\theta$ darstellt und
       - $R_{\theta'}(435\,nm)$ den Wert der Reflektivität der den Filter beinhaltenden Hauptseite bei der Wellenlänge von 435 Nanometern beim Einfallswinkel $\theta'$ darstellt.

2. Ophthalmische Linse nach Anspruch 1, bei welcher der Filter ein Reflexionsfilter ist, der eine ausgezeichnete Transmission im Wellenlängenbereich von 465 bis 495 nm ermöglicht.

3. Ophthalmische Linse nach einem der Ansprüche 1 und 2, bei der das Mittel zum Sperren des auf die vordere Hauptseite treffenden UV-Lichts 90 % oder mehr des UV-Lichts sperrt.

4. Ophthalmische Linse nach einem der Ansprüche 1 bis 3, bei welcher der Filter, der das blaue Licht zumindest teilweise blockiert, auf der vorderen Hauptseite der ophthalmischen Linse gebildet ist.

5. Ophthalmische Linse nach einem der vorhergehenden Ansprüche, bei welcher der Filter, der das blaue Licht blockiert, 5 bis 50 % des blauen Lichts im Wellenlängenbereich von 420 bis 450 Nanometer blockiert.

6. Ophthalmische Linse nach einem der vorhergehenden Ansprüche, bei welcher der Filter der Hauptseite der den Filter umfassenden ophthalmischen Linse die Eigenschaft verleiht, bei einem Einfallswinkel auf dieser Hauptseite zwischen 0° und 15° einen mittleren Reflexionsfaktor im Blaubereich $R_{m,B}$ aufzuweisen, der größer oder gleich 5 % ist.

7. Ophthalmische Linse nach einem der vorhergehenden Ansprüche, bei welcher der Einfallswinkel $\theta = 15°$ und der Einfallswinkel $\theta' = 45°$ ist.

8. Ophthalmische Linse nach einem der vorhergehenden Ansprüche, bei welcher der das blaue Licht blockierende Filter der den Filter beinhaltenden Hauptseite ferner die folgende Eigenschaft verleiht:

- eine Kurve der spektralen Reflektivität bei einem Einfallswinkel zwischen 0° und 15°, die Folgendes aufweist:

o ein Reflektivitätsmaximum bei einer Wellenlänge unter 435 Nanometer und
o eine Halbwertsbreite (FWHM) größer oder gleich 70 nm, bevorzugt größer oder gleich 75 nm und besser größer oder gleich 80 nm und kleiner als 150 nm, bevorzugt kleiner oder gleich 120 nm und besser kleiner oder gleich 110 nm.

9. Ophthalmische Linse nach Anspruch 8, bei der das Reflektivitätsmaximum bei einer Wellenlänge kleiner oder gleich 400 nm, besser kleiner oder gleich 390 nm liegt.

10. Ophthalmische Linse nach Anspruch 8 oder 9, bei der das Reflektivitätsmaximum bei einer Wellenlänge größer oder gleich 350 nm liegt.

11. Ophthalmische Linse nach einem der vorhergehenden Ansprüche, bei welcher der mittlere Lichtreflexionsfaktor ($R_v$) auf der den Filter beinhaltenden Hauptseite der ophthalmischen Linse kleiner oder gleich 2,5 % ist.

12. Ophthalmische Linse nach einem der vorhergehenden Ansprüche, bei welcher der mittlere Lichtreflexionsfaktor ($R_v$) auf jeder der Hauptseiten der ophthalmischen Linse kleiner oder gleich 2,5 %, bevorzugt kleiner oder gleich 1,5 % ist.

13. Ophthalmische Linse nach einem der vorhergehenden Ansprüche, bei welcher der gewichtete mittlere Reflexionsfaktor im UV-Bereich der Entspiegelung auf der hinteren Hauptseite kleiner oder gleich 6 %, bevorzugt kleiner oder gleich 5 %, besser kleiner oder gleich 4,5 % und idealerweise kleiner oder gleich 3,5 % ist.

14. Ophthalmische Linse nach einem der vorhergehenden Ansprüche, bei welcher der Filter ein Interferenzfilter ist.

15. Ophthalmische Linse nach einem der vorhergehenden Ansprüche, bei der das Verhältnis $\Delta$ spektral @ 15° = $[R_{15°}$ (435 nm) - $R_{15°}$ (480 nm)]/$R_{15°}$ (435 nm), wobei $R_{15°}$ (435 nm) und $R_{15°}$ (480 nm) jeweils die Reflektivität der den Filter beinhaltenden Hauptseite der ophthalmischen Linse bei einer Wellenlänge von 435 nm und bei einer Wellenlänge von 480 nm bei einem Einfallswinkel auf dieser Hauptseite von 15° darstellen, größer oder gleich 0,8, besser größer oder gleich 0,85 und noch besser größer oder gleich 0,9 ist.

16. Brillenglas, das aus einer ophthalmischen Linse nach einem der vorhergehenden Ansprüche besteht, **dadurch gekennzeichnet, dass** es unter den weißen Gläsern, den behandelten Gläsern, den Sonnenschutzgläsern und den photochromen Gläsern gewählt ist.

**Claims**

1. An ophthalmic lens having a front main face and a back main face comprising:

- a means for cutting off ultraviolet (UV) light arriving on the front main face of the ophthalmic lens, said cutting means being made of a substrate of the ophthalmic lens;

- an antireflection coating, on the back main face of the ophthalmic lens, having an average reflection factor in the UV weighted by the function $M(\lambda)$ defined in the standard ISO 13666:1998 lower than or equal to 7%; and
- at least one filter blocking at least partially the blue light in the wavelength range extending from 420 to 450 nanometers, said filter being formed on a main face of the lens and **characterized in that** it provides the main face comprising said filter with the following property:

- for an angle of incidence $\theta$ ranging from 0° to 15° and for an angle of incidence $\theta'$ ranging from 30° to 45° , a parameter $\Delta(\theta,\theta')$ defined by the relation $\Delta(\theta,\theta') = 1 - [R_{\theta'}(435\,nm) / R_{\theta}(435\,nm)]$, so that this parameter $\Delta(\theta,\theta')$ is higher than or equal to 0.5 and preferably higher than or equal to 0.6, where

- $R_{\theta}(435\,nm)$ represents the reflectivity value of the main face comprising said filter at a 435 nanometer-wavelength for the angle of incidence $\theta$, and
- $R_{\theta'}(435\,nm)$ represents the reflectivity value of the main face comprising said filter at a 435 nanometer-wavelength for the angle of incidence $\theta'$.

2. The ophthalmic lens as claimed in claim 1, wherein said filter is a reflection filter allowing an outstanding transmission within the wavelength range of from 465 nm to 495 nm

3. The ophthalmic lens as claimed in claim 1 or 2, wherein the means for cutting off UV light arriving on the front main face cuts off 90% or more of the UV light.

4. The ophthalmic lens as claimed in any one of claims 1 to 3, wherein said filter blocking at least partially the blue light is formed on the front main face of the ophthalmic lens.

5. The ophthalmic lens as claimed in any one of the preceding claims, wherein the filter blocking the blue light blocks from 5 to 50% of the blue light in the wavelength range from 420 to 450 nanometers.

6. The ophthalmic lens as claimed in any one of the preceding claims, wherein said filter provides the main face of the ophthalmic lens comprising said filter with the property of having, for an angle of incidence on this main face ranging from 0° to 15°, an average blue reflectance factor $R_{m,B}$ which is higher than or equal to 5%.

7. The ophthalmic lens as claimed in any one of the preceding claims, wherein the angle of incidence $\theta$ = 15° and the angle of incidence $\theta'$ = 45°.

8. The ophthalmic lens as claimed in any one of the preceding claims, wherein the blue-light blocking filter furthermore provides the main face comprising said filter with the following property:

- a spectral reflectivity curve for an angle of incidence ranging from 0° to 15° having:

- a reflectivity maximum at a wavelength lower than 435 nanometers; and
- a full width at half maximum (FWHM) higher than or equal to 70 nm, preferably higher than or equal to 75 nm and better still higher than or equal to 80 nm and lower than 150 nm, preferably lower than or equal to 120 nm and better still lower than or equal to 110 nm.

9. The ophthalmic lens as claimed in claim 8, wherein said reflectivity maximum is at a wavelength lower than or equal to 400 nm, better lower than or equal to 390 nm.

10. The ophthalmic lens as claimed in claim 8 or 9, wherein said reflectivity maximum is at a wavelength higher than or equal to 350 nm.

11. The ophthalmic lens as claimed in any one of the preceding claims, wherein the average light reflection factor ($R_v$) on the main face of the ophthalmic lens comprising said filter is lower than or equal to 2.5%.

12. The ophthalmic lens as claimed in any one of the preceding claims, wherein the average light reflection factor ($R_v$) on each of the main faces of the ophthalmic lens is lower than or equal to 2.5% and preferably lower than or equal to 1.5%.

13. The ophthalmic lens as claimed in any one of the preceding claims, wherein the weighted average reflection factor in the UV of the antireflection coating on the back main face is lower than or equal to 6%, preferably lower than or equal to 5%, better still lower than or equal to 4.5% and ideally lower than or equal to 3.5%.

14. The ophthalmic lens as claimed in any one of the preceding claims, wherein the filter is an interference filter.

15. The ophthalmic lens as claimed in any one of the preceding claims, wherein the ratio $\Delta_{spectral@15°} = [R_{15°}(435nm)-R_{15°}(480nm)]/R_{15°}(435nm)]$, where $R_{15°}(435nm)$ and $R_{15°}(480nm)$ represent respectively the reflectivity value of the main face of the ophthalmic lens at a wavelength of 435 nm and at a wavelength of 480 nm, for an angle of incidence on said main face of 15°, higher than or equal to 0.8, better higher than or equal to 0.85, better still higher than or equal to 0.9.

16. Spectacle glass formed from an ophthalmic lens according to any one of the preceding claims, wherein it is chosen from clear glasses, processed glasses, sunglasses and photochromic glasses.

Figure 1

Figure 2

Figure 3

Figure 4

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2012075577 A
- WO 2008024414 A
- US 8360574 B
- WO 2012076714 A1
- FR 2734827
- EP 2011072386 W
- EP 0614957 A
- US 7678464 B
- EP 614957 A
- WO 2008107325 A

**Littérature non-brevet citée dans la description**

- **KITCHEL E.** The effects of blue light on ocular health. *Journal of Visual Impairment and Blindness,* 2000, vol. 94 (6
- **GLAZER-HOCKSTEIN et al.** *Retina,* 2006, vol. 26 (1), 1-4
- **ALGVERE P.V. et al.** Age-Related Maculopathy and the Impact of the Blue Light Hazard. *Acta Ophthalmo. Scand.,* 2006, vol. 84, 4-15
- **TOMANY S.C. et al.** Sunlight and the 10-Year Incidence of Age-Related Maculopathy. The Beaver Dam Eye Study. *Arch Ophthalmol.,* 2004, vol. 122, 750-757
- **CITEK.** *K Optometry,* 2008, vol. 79, 143-148